(19) Europäisches Patentamt
European Patent Office
Office européen des brevets

(11) Veröffentlichungsnummer: **0 409 119 B1**

# (12) EUROPÄISCHE PATENTSCHRIFT

(45) Veröffentlichungstag der Patentschrift: **13.04.94**

(51) Int. Cl.[5]: **C07D 213/81**, A61K 31/44

(21) Anmeldenummer: **90113528.5**

(22) Anmeldetag: **14.07.90**

Die Akte enthält technische Angaben, die nach dem Eingang der Anmeldung eingereicht wurden und die nicht in dieser Patentschrift enthalten sind.

## (54) N,N'-Bis(alkoxy-alkyl)-pyridin-2,4-dicarbonsäurediamide, Verfahren zu deren Herstellung sowie deren Verwendung.

(30) Priorität: **20.07.89 DE 3924093**

(43) Veröffentlichungstag der Anmeldung:
**23.01.91 Patentblatt 91/04**

(45) Bekanntmachung des Hinweises auf die Patenterteilung:
**13.04.94 Patentblatt 94/15**

(84) Benannte Vertragsstaaten:
**AT BE CH DE DK ES FR GB GR IT LI LU NL SE**

(56) Entgegenhaltungen:
**EP-A- 0 278 453**

**CHEMICAL ABSTRACTS, Band 55, Nr 11, 29 Mai 1961, Spalte 10439g-10440e, Columbus, Ohio, US; S.Samejima & Yagugaku Zasshi 1960, Band 80, Seiten 1706-1712**

(73) Patentinhaber: **HOECHST AKTIENGESELLSCHAFT**

**D-65926 Frankfurt(DE)**

(72) Erfinder: **Baader, Ekkehard, Dr.**
**Amselweg 14**
**D-6240 Königstein/Taunus(DE)**
Erfinder: **Bickel, Martin, Dr.**
**Mittelstedter Weg 3**
**D-6380 Bad Homburg(DE)**
Erfinder: **Günzler-Pukall, Volkmar, Dr.**
**Gross-Seelheimer Strasse 13**
**D-3550 Marburg(DE)**

EP 0 409 119 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Verbindungen die die Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren, wie $\alpha,\alpha'$-Dipyridyl zu einer Hemmung der $C1_q$-Biosynthese von Makrophagen führt (W. Müller et al., FEBS Lett. 90 (1978), 218; Immunbiology 155 (1978) 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß Prolinhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238, 625-633, 1987).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäure, die die Kollagenbiosynthese im Tiermodell wirksam hemmen.

So wird in der DE-A 37 03 959 unter anderem die Synthese von N,N'-Bis(2-methoxyethyl)-pyridin-2,4-dicarbonsäurediamid(III)

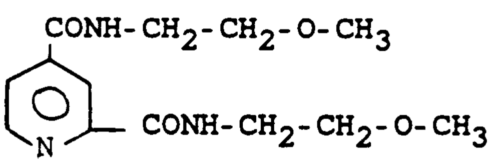

(III)

und

N,N'-Bis(3-isopropoxypropyl)-pyridin-2,4-dicarbonsäurediamid(IV)

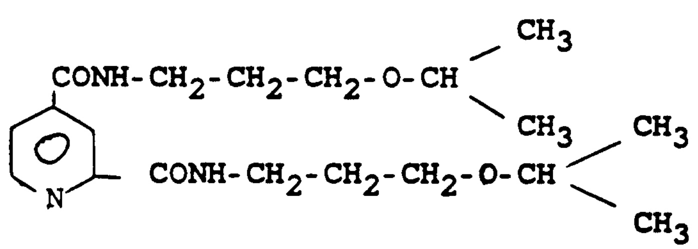

(IV)

beschrieben.

In den deutschen Patentanmeldungen P 38 26 471.4 und P 38 28 140.6 wird ein verbessertes Verfahren zur Herstellung von N,N'-Bis(2-methoxyethyl)-pyridin-2,4-dicarbonsäurediamid (III) vorgeschlagen.

Die enterale Resorbierbarkeit vieler der in der DE-A 37 03 959 beschriebenen Verbindungen ist jedoch noch unbefriedigend, so daß ein Bedürfnis bestand, Verbindungen zur Verfügung zu stellen, die nach oraler Gabe schon bei niedrigen Dosierungen die Prolin- und Lysinhydroxylase wirksam hemmen.

Es wurde nun gefunden, daß Verbindungen der Formel I

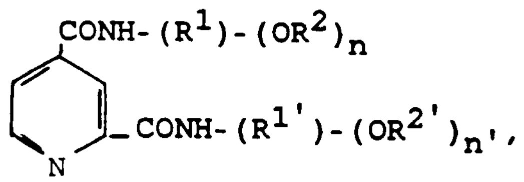

(I)

worin

| | |
|---|---|
| $R^1$ | lineares oder verzweigtes $C_1$-$C_4$-Alkandiyl, |
| $R^2$ | unverzweigtes $C_1$-$C_4$-Alkyl oder Wasserstoff n 1 oder 2 bedeutet und |
| $R^{1'}$, $R^{2'}$ und n' | die selben Bedeutungen wie $R^1$, $R^2$ und n haben, wobei $R^1$ und $R^{1'}$, und $R^2$ und $R^{2'}$ und n und n' identisch oder verschieden sind, |

sowie deren physiologisch verträgliche Salze, ausgenommen N,N'-Bis(2-methoxyethyl)-pyridin-2,4-dicarbonsäurediamid und N,N'-Bis(2-hydroxyethyl)-pyridin-2,4-dicarbonsäurediamid, die oben genannte Aufgabe erfüllen.

Im Vergleich zu den in der DE-A 37 03 959 beschrieben Verbindungen N,N'-Bis(2-methoxyethyl)-pyridin-2,4-dicarbonsäurediamid und N,N'-Bis(3-isopropoxypropyl)-pyridin-2,4-dicarbonsäurediamid weisen die Verbindungen der Formel I sowohl eine bessere pharmakologische Wirksamkeit als auch eine bessere enterale Resorbierbarkeit auf.

Weiterhin betrifft die Erfindung ein Verfahren zur Herstellung von N,N'-Bis-(alkoxyalkyl)-pyridin-2,4-dicarbonsäurediamiden der Formel I

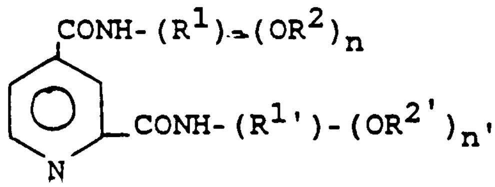

(I)

worin

| | |
|---|---|
| $R^1$ | lineares oder verzweigtes $C_1$-$C_4$ Alkandiyl, |
| $R^2$ | unverzweigtes $C_1$-$C_4$-Alkyl oder Wasserstoff |
| n | 1 oder 2 bedeutet und |
| $R^{1'}$, $R^{2'}$ und n' | die selben Bedeutungen wie $R^1$, $R^2$ und n haben, wobei $R^1$ und $R^{1'}$, $R^2$ und $R^{2'}$ und n und n' identisch oder verschieden sind, ausgenommen N,N'-Bis(2-methoxyethyl)-pyridin-2,4-dicarbonsäurediamid und N,N' Bis(2-hydroxyethyl)-pyridin-2,4-dicarbonsäurediamid, |

das dadurch gekennzeichnet ist, daß man Pyridin-2,4-dicarbonsäurehalogenid mit einem Alkoxyalkylamin oder Hydroxylalkylamin umsetzt.

Die Erfindung betrifft ebenfalls ein Verfahren zur Herstellung von N,N'-Bis-(alkoxyalkyl)-pyridin-2,4-dicarbonsäurediamiden der Formel I

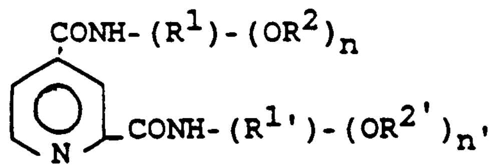

(I)

worin

| | |
|---|---|
| $R^1$ | lineares oder verzweigtes $C_1$-$C_4$-Alkandiyl, |
| $R^2$ | unverzweigtes $C_1$-$C_4$-Alkyl oder Wasserstoff |
| n | 1 oder 2 bedeutet und |

| | |
|---|---|
| $R^{1'}$, $R^{2'}$ und n′ | dieselben Bedeutungen wie $R^1$, $R^2$ und n haben, wobei $R^1$ und $R^{1'}$, $R^2$ und $R^{2'}$ und n und n′ identisch oder verschieden sind, |

das dadurch gekennzeichnet ist, daß man zunächst

A)

1. Pyridin-2,4-dicarbonsäure mit mindestens zwei Äquivalenten eines Halogenierungsmittels versetzt, und daß man

2. mindestens 2 Äquivalente Hydroxyalkylamin oder Alkoxyalkylamin der Formel II oder II′

$$H_2N\text{-}(R^1)\text{-}(OR^2)n \quad (II)$$

$$H_2N\text{-}(R^{1'})\text{-}(OR^{2'})_{n'} \quad (II')$$

worin

| | |
|---|---|
| $R^1$ und $R^{1'}$ | lineares oder verzweigtes $C_1$-$C_4$-Alkandiyl, |
| $R^2$ und $R^{2'}$ | unverzweigtes $C_1$-$C_4$-Alkyl oder Wasserstoff und |
| n und n′ | 1 oder 2 bedeutet und |
| $R^1$ und $R^{1'}$, $R^2$ und $R^{2'}$ | sowie n und n′ identisch oder verschieden sind, wobei aber II und II′ verschieden sind, |

in einem Lösungsmittel löst

und daß man dann die gemäß 1. hergestellte Lösung mit der gemäß 2. hergestellten Lösung zur Reaktion bringt, oder daß man

B) das so erhaltene N,N′-Bis(alkoxyalkyl)-pyridin-2,4-dicarbonsäurediamid in die Bis(hydroxyalkyl)-verbindung überführt oder das man

C) das gemäß A) 1. hergestellte Pyridin-2,4-dicarbonsäurehalogenid mit einem substituierten oder unsubstituierten Benzylalkohol zum Pyridin-2,4-dicarbonsäurebenzylester umsetzt, den Benzylester selektiv in 2-Position des Pyridins verseift, gemäß Verfahrensvariante A) 1. die freie Carbonsäure in 2-Position erneut in das Säurehalogenid überführt und die so erhaltene Verbindung mit einer gemäß A) 2. hergestellten Lösung unter Verwendung einer Verbindung der Formel II′ versetzt, wobei ein Pyridin-4-carbonsäurebenzylester-2-carbonsäureamid entsteht, und daß man anschließend die Benzylschutzgruppe in 4-position hydrogenolytisch abspaltet, die freie Carbonsäure gemäß Verfahrensvariante A) 1. erneut in das Säurechlorid überführt, welches dann mit einer gemäß A) 2. hergestellten Lösung unter Verwendung einer Verbindung der Formel II versetzt wird, wobei eine unsymmetrisch substituierte Verbindung der Formel I entsteht und daß man ggf. anschließend die erhaltene Verbindung der Formel I in ihr physiologisch verträgliches Salz überführt.

Bei dem Verfahren zur Herstellung der Verbindungen der Formel I wird die als Ausgangssubstanz käuflich erhältliche Pyridin-2,4-dicarbonsäure in einem Lösungsmittel wie Toluol suspendiert und bei Raumtemperatur mit einem Halogenierungsmittel, bevorzugt einem Chlorierungsmittel wie z.B. $SOCl_2$, versetzt. Bezogen auf die eingesetzte molare Menge an Pyridin-2,4-dicarbonsäure werden 2-3 Äquivalente eines Halogenierungsmittels eingesetzt, bevorzugt 2,5 Äquivalente. Die erhaltene Reaktionsmischung wird auf 90-110 °C, vorzugsweise auf 100 °C, solange erhitzt, bis keine Gasentwicklung mehr zu beobachten ist und eine klare Lösung entstanden ist. Anschließend wird von der Lösung -vorzugsweise im Hochvakuum (bis ca. $10^{-3}$ Torr)-10 % eingedampft, und das erhaltene Carbonsäurehalogenid weiter umgesetzt.

Bezogen auf die eingesetzte molare Menge an Pyridin-2,4-dicarbonsäure wird nun die 2-4-fach molare Menge an käuflichem Alkoxyalkylamin oder Hydroxyalkylamin in einem Lösungsmittel wie Toluol gelöst und bevorzugt die 2-4-fach molare Menge einer Base wie Triethylamin hinzugegeben. Das Carbonsäurehalogenid wird mit dem Alkoxyalkylamin bzw. dem Hydroxylalkylamin zur Reaktion gebracht. Dies geschieht bevorzugt indem man die Lösung des genannten -alkylamins zu dem gelösten Pyridin-2,4-dicarbonsäurehalogenid tropft. Es ist jedoch auch möglich, die Lösung des Carbonsäurehalogenids zu der Lösung des Alkoxyalkylamins bzw. Hydroxalkylamins zu tropfen. Die Zugabe erfolgt bei einer Temperatur von -5 bis +5 °C, bevorzugt bei 0 °C. Die Reaktionsmischung läßt man dann nachreagieren indem man z.B. auf Raumtemperatur erwärmt und noch 2-5 Stunden, bevorzugt 3 Stunden nachrührt. Das erhaltene Produkt wird anschließend angesäuert, um überschüssiges Hydroxy- bzw. Alkoxyalkylamin vom gewünschten Produkt abzutrennen. Das Ansteuern kann beispielsweise mit 0,2 molarer Citronensäure erfolgen. Die organische Phase wird dann abgetrennt und mit Wasser gewaschen. Anschließend wird die organische Phase getrocknet -vorzugsweise über Magnesiumsulfat- und schließlich vom Lösungsmittel befreit. Beim Entfernen des Lösungsmittels fällt das Produkt als weißer Festkörper oder als Öl an.

Zur Herstellung der N,N′-Bis(hydroxyalkyl)-pyridin-2,4-dicarbonsäurediamide geht man am besten so vor, daß man ein entsprechendes Bis(alkoxyalkyl)diamid, bevorzugt Bis(methoxyalkyl)diamid, nach literaturbekannten Verfahren, beispielsweise mit Bortribromid, in das entsprechende Bis(hydroxyalkyl)diamid überführt.

Unsymmetrisch substituierte Verbindungen der Formel I können beispielsweise folgendermaßen synthetisiert werden:
Umsetzung eines Pyridin-2,4-dicarbonsäurehalogenids, vorzugsweise des -chlorids, mit substituiertem oder unsubstituiertem Benzylalkohol zum Pyridin-2,4-dicarbonsäurebenzylester, anschließende selektive Verseifung des Esters in 2-Position (z.B. in Gegenwart eines Kupferkatalysators, Acta Helv. 44, 1963, S. 637), Überführung der freien Saure in 2-Stellung in das Säurehalogenid, Umsetzung mit einer Verbindung der Formel (II') zum Pyridin-4-carbonsäurebenzylester-2-carbonsäureamid, hydrogenolytischer Abspaltung der verbliebenen Benzylschutzgruppe (z.B. mit $H_2$/Pd, s. Houben-Weyl Bd. IV/1c (1980), s. 381-82) und anschließender Überführung der freien Säure in 4 Position des Pyridinrings in das Säurehalogenid.
Das Säurehalogenid kann nun mit einem Amin II in das gemischte Diamid (I) überführt werden (siehe Reaktionsschema).

Gegebenenfalls kann die Aufarbeitung der Produkte beispielsweise durch Extraktion oder durch Chromatographie z.B. über Kieselgel erfolgen. Das isolierte Produkt kann umkristallisiert und gegebenenfalls mit einer geeigneten Saure zu einem physiologisch verträglichen Salz umgesetzt werden. Als geeignete Säuren kommen beispielsweise in Betracht:
Mineralsäuren, wie Chlorwasserstoff- und Bromwasserstoffsäure sowie Schwefel-, Phosphor-, Salpeter- oder Perchlorsäure oder organische Säuren wie Ameisen-, Essig-, Propion-, Bernstein-, Glykol-, Milch-, Äpfel-, Wein-, Zitronen-, Malein-, Fumar-, Phenylessig- Benzoe-, Methansulfon-, Toluolsulfon-, Oxal-, 4-Aminobenzoe-, Naphthalin-1,5-disulfon- oder Ascorbinsäure.

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Die pharmazeutischen Präparate können in fester Form, z.B. als Tabletten, Dragees, Suppositiorien oder Kapseln; in halbfester Form, z.B. als Salben, oder in flüssiger Form, z.B. als Lösungen, Suspensionen oder Emulsionen, vorliegen. Gegebenenfalls sind sie sterilisiert und/oder enthalten Hilfsstoffe, wie Konservierungs-, Stabilisierungs-, Netz- oder Emulgiermittel, Salze zur Veränderung des osmotischen Drucks oder Puffer. Sie können auch noch andere therapeutisch wirksame Stoffe enthalten.

Es wurde festgestellt, daß die Verbindungen der Formel I außergewöhnlich gute enterale Resorbierbarkeiten aufweisen. Die Resorbierbarkeit wurde an Wistarratten untersucht, denen die erfindungsgemäßen Verbindungen intragastral verabreicht wurden. Der Serumspiegel sank in den ersten Stunden nach Verabreichung der Substanz ab und erreichte nach ca. 5 Stunden ein nur noch leicht abfallendes Plateau. Aus dem zunächst sehr hohen Serumspiegel direkt nach Verabreichung der Substanzen läßt sich auf die gute Resorbierbarkeit der Substanzen schließen.

Im folgenden ist die Erfindung anhand von Beispielen näher erläutert.

**Beispiel 1**

Pyridin-2,4-dicarbonsäure-bis N,N'-(methoxypropyl)amid

3 g Pyridin-2,4-dicarbonsäure werden in 50 ml Toluol und 1 ml DMF vorgelegt und zur Lösung 2,7 ml Thionylchlorid zugetropft. Es wird solange erhitzt, bis keine Gasentwicklung mehr zu beobachten ist (ca. 2,5 h). Es wird abgekühlt, 5 ml Toluol abdestilliert und zur Lösung 4,6 ml 3-Methoxypropylamin und 5 ml Triethylamin zugetropft. Nach Rühren der Lösung bei Raumtemperatur für 4 h wird eingedampft, der Rückstand mit Wasser aufgenommen und 4 x mit Methylenchlorid extrahiert. Die vereinigten organischen Phasen werden über Magnesiumsulfat getrocknet und eingedampft. Das Rohprodukt wird mit Kieselgel (Lösungsmittel Ethylacetat) chromatographiert.
Ausbeute: 4,3 g; Öl

$^1$H-NMR ($CDCl_3$): $\delta$ = 1,6-2,3 (4H, m); 3,2-3,8 (14H, m); 7,8-8,0 (1H, m); 8,3-8,5 (1H, m); 8,6-8,8 (1H, m).

**Beispiel 2**

Pyridin-2,4-dicarbonsäure-bis-N,N′-(ethoxypropyl)amid

Vorschrift siehe Beispiel 1; Aminkomponente Ethoxypropylamin,
Ausbeute: 4,5 g, Fp.: 46-48 °C.

$^1$H-NMR ($CDCl_3$): $\delta$ = 1,3 (6H, tr); 1,7-2,1 (4H, m); 3,3-3,8 (12H, m); 7,8-8,0 (1H, m); 8,4-8,5 (1H, m); 8,5-8,8 (1H, m);

**Beispiel 3**

Pyridin-2,4-dicarbonsäure-bis-N,N′-(2-dimethoxyethyl)amid

Vorschrift siehe Beispiel 1, Aminkomponente 2-Dimethoxyethylamin,
Ausbeute: 1,6 g (aus 3 g Pyridin-2,4-dicarbonsäure), Öl
$^1$H-NMR ($CDCl_3$): $\delta$ = 3,4 (12H, s); 3,7 (4H, m); 4,5 (2H, m); 7,9-8,0 (1H, m); 8,4-8,5 (1H, m); 8,7-8,8 (1H, m);

**Beispiel 4**

Pyridin-2,4-dicarbonsäure-bis-N,N′-(2-methoxyisopropyl)amid

Vorschrift siehe Beispiel 1; Aminkomponente 2-Methoxyisopropylamin;
Ausbeute: 3,3 g (aus 3 g Pyridin-2,4-dicarbonsäure), Öl
$^1$H-NMR ($CDCl_3$): $\delta$ = 1,3 (6H, d); 3,2 (6H, s); 3,5 (4H, d); 4,4 (2H, m); 7,9-8,0 (1H, m); 8,4-8,5 (1H, m); 8,7-8,8 (1H, m);

**Beispiel 5**

Pyridin-2,4-dicarbonsäure-bis-N,N′-(2-ethoxyethyl)amid

Vorschrift siehe Beispiel 1; Aminkomponente Ethoxyethylamin,
Ausbeute 7,8 g (aus 10 g Pyridin-2,4-dicarbonsäure),
Fp.: 42-44 °C
$^1$H-NMR ($CDCl_3$): $\delta$ = 1,2 (3H, tr); 3,3-3,8 (12H, qu. und m); 7,9 (1H, m); 8,4-8,5 (1H, m); 8,7-8,8 (1H, m);

**Beispiel 6**

Pyridin-2,4-dicarbonsäure-bis-N,N′-(3-hydroxy-ethyl)amid

0,5 g Pyridin-2,4-dicarbonsäure-bis-N,N′-(3-methoxyethyl) amid werden in 10 ml Dichlormethan gelöst und bei -78 °C Bortribromid (11 ml, 1 molare Lösung in Dichlormethan) zugetropft. Nach beendeter Zugabe auf Raumtemperatur kommen lassen und 3 Stunden nachrühren. Auf 100 ml gesättigte Bicarbonat-Lösung gießen und 3 x mit Ethylacetat extrahieren. Die vereinigten organischen Lösungsmittel werden mit Magnesiumsulfat getrocknet und eingedampft. Das Rohprodukt wird an Kieselgel chromatographiert.
Ausbeute 0,45 g; Öl
$^1$H-NMR ($CDCl_3$): $\delta$ = 1,5-2,2 (4H,m); 3,4 (4H, m); 3,6 (4H,m); 7,9-8,0 (1H, m); 8,4-8,5 (1H, m); 8,7-8,8 (1H, m);

**Beispiel 7a**

Pyridin-2,4-dicarbonsäuredibenzylester

30 g Pyridin-2,4-dicarbonsäure werden analog Beispiel 1 mit 30 ml Thionylchlorid ins Säurechlorid überführt und mit 43,8 g Benzylalkohol umgesetzt. Das Produkt wird aus Diisopropylether umkristallisiert.
Ausbeute: 42,1 g Schmelzpunkt 63-65 °C

**Beispiel 7b**

Pyridin-2-carbonsäure-4-carbonsäurebenzylester

40 g Pyridin-2,4-dicarbonsäuredibenzylester aus Beispiel 7a werden zu einer Suspension aus 27,8 g Kupfer-II-nitrat in 700 ml Methanol gegeben. Es wird eine Stunde unter Rückfluß gekocht und nach dem Abkühlen vom Kupferkomplex abfiltriert. Der Komplex wird in Dioxan suspendiert und Schwefelkohlenstoff eingeleitet. Das ausgefallene Kupfersulfid wird abfiltriert und die organische Phase eingeengt. Das Produkt wird mit Petrolether verrührt.
Ausbeute 25,3 g Schmelzpunkt 113-115 °C

**Beispiel 7c**

Pyridin-2-(3-methoxypropyl)-carbonsäureamid-4-carbonsäurebenzylester

3,9 g Pyridin-2-carbonsäure-4-carbonsäurebenzylester aus Beispiel 7b werden analog Beispiel 1 mit 1,2 ml Thionylchlorid in das Säurechlorid überführt und mit 3-Methoxypropylamin zum Amid umgesetzt. Das Produkt wird zur Reinigung über Kieselgel mit einer Mischung aus Cyclohexan/Ethylacetat (1:1) chromatographiert.
Ausbeute 4,3 g Öl

**Beispiel 7d**

Pyridin-4-carbonsäure-2-(3-methoxypropyl)-carbonsäureamid

4,3 g Verbindung aus Beispiel 7c werden in 100 ml Dioxan gelöst und mit 500 mg Palladium/Kohle (10 %)-Katalysator unter Normaldruck für 4 Stunden hydriert. Nach Beendigung der Wasserstoffaufnahme wird vom Katalysator abgesaugt und vom Lösungsmittel abgezogen.
Ausbeute: 3,5 g Schmelzpunkt 124-126 °C

**Beispiel 7e**

Pyridin-4-carbonsäure-(2-methoxyethyl)-2-carbonsäure-(3-methoxypropyl)-diamid

Entsprechend Beispiel 1 werden 1,8 g Verbindung aus Beispiel 7d mit 0,6 ml Thionylchlorid in das Säurechlorid überführt und anschließend mit 2-Methoxyethylamin umgesetzt. Das Produkt wird zur Reinigung über Kieselgel mit einer Mischung aus Dichlormethan/Methanol (20:1) chromatographiert.
Ausbeute: 1,0 g Öl
$^1$H-NMR ($CDCl_3$): $\delta$ = 1,9-2,0 (2H, qui); 3,4 (6H,s); 3,5-3,7 (8H, m); 6,9 (1H, s, br); 8,0 (1H, dd); 8,4 (1H, s, br); 8,5 (1H, s); 8,7 (1H, d);

**Beispiel 8**

Pyridin-2-carbonsäure-(2-methoxyethyl)-4-carbonsäure-(3-methoxypropyl)-diamid

Analog zu den Beispielen 7a-e wird die Verbindung gemäß Beispiel 8 hergestellt, indem im Reaktionsschritt Beispiel 7c 2-Methoxyethylamin und im Reaktionsschritt Beispiel 7e 3-Methoxypropylamin eingesetzt wird.
Schmelzpunkt: 69-72 °C
$^1$H-NMR ($CDCl_3$): $\delta$ = 1,9-2,0 (2H, qui); 3,4 (3H, s); 3,45 (3H, s); 3,6-3,7 (8H, m); 7,4 (1H, br); 7,9 (1H, dd); 8,3 (1H, br); 8,4 (1H, d); 8,7 (1H, d);

**Beispiel 9**

Enterale Resorbierbarkeit

Weibliche Wistarratten von ca. 150 g Körpergewicht erhalten 50 mg/kg der zu untersuchenden Substanz mittels Schlundsonde intragastral verabreicht. Es werden nach 5; 10; 15; 30; 60; 120; 180 und 240

Minuten jeweils 4 Ratten narkotisiert und über die Vena Cava entblutet. Das Blut wird sofort zentrifugiert und die verabreichte Verbindung mit Ether aus dem Serum extrahiert. Nach Verdampfen des Ethers wird der Rückstand in 100 ml Fließmittel aufgenommen. Das Fließmittel besteht aus 0,05 M Phosphorsäure und Acetonitril (4:1).
Von dieser Probe werden 50 µl in eine HPLC-Säule injiziert. Die Detektion erfolgt bei UV 200 nm und einer Retentionszeit von 2,2 min. Die Ergebnisse sind in der Tabelle 1 dokumentiert.

**Tabelle 1:** Serumspiegel der erfindungsgemäßen Verbindungen aus den Beispielen 1-3 nach Gabe von 50 mg/kg p.o.

| Zeit (Min). | Substanz aus Beispiel 1 $\bar{x}$ | SD | Substanz aus Beispiel 2 $\bar{x}$ | SD | Substanz aus Beispiel 3 $\bar{x}$ | SD |
|---|---|---|---|---|---|---|
| 5 | 45.3 | ± 15.4 | 51.4 | ± 11.2 | 8.9 | ± 3.1 |
| 10 | 49.8 | ± 3.6 | 39.2 | ± 4.0 | 11.5 | ± 0.6 |
| 15 | 39.9 | ± 11.0 | 29.4 | ± 6.7 | 14.7 | ± 1.9 |
| 30 | 28.1 | ± 3.2 | 15.2 | ± 5.6 | 10.7 | ± 1.9 |
| 60 | 9.4 | ± 5.5 | 1.4 | ± 1.0 | 11.3 | ± 1.5 |
| 120 | 0.3 | ± 0.3 | <NWG | | 5.5 | ± 0.9 |
| 180 | <NWG | | <NWG | | 2.9 | ± 0.5 |
| 240 | <MWG | | <NWG | | 1.7 | ± 0.4 |

$\bar{x}$ = Mittelwert aus 4 Messungen

SD = Standardabweichung

<NWG = unterhalb der Nachweisgrenze

**Beispiel 10**

Pharmakologische Wirksamkeit

Zum Nachweis der effektiven Hemmung der Prolin- und Lysinhydroxylase durch die erfindungsgemäßen Verbindungen wurden die Hydroxyprolin Konzentrationen in der Leber und die 7s-(IV)-Kollagen-Konzentrationen im Serum von

a) unbehandelten Ratten (Kontrolle)

b) Ratten, denen Tetrachlorkohlenstoff verabreicht wurde ($CCl_4$-Kontrolle)

c) Ratten, denen zunächst $CCl_4$ und anschließend eine erfindungsgemaße Verbindung verabreicht wurde

gemessen (diese Testmethode wird beschrieben von Rouiller, C., experimental toxic injury of the liver; in The Liver, C. Rouiller, Vol. 2, S. 335-476, New York, Academic Press, 1964).

Die Wirkstärke der erfindungsgemäßen Verbindungen wurde als prozentuale Hemmung der Leber Hydroxyprolin- und Serum-7s-(IV)-kollagensynthese nach oraler Gabe im Vergleich zu Kontrolltieren, denen nur Tetrachlorkohlenstoff verabreicht wurde ($CCl_4$-Kontrolle), bestimmt. Die Ergebnisse sind in Tabelle 2 aufgeführt. Als Vergleichssubstanzen sind die Verbindungen aus den Beispielen 2 und 3 der DE-A 37 03 959 (N,N′-Bis(2-methoxyethyl)-pyridin-2,4-dicarbonsäurediamid und N,N′-Bis(3-isopropoxypropyl)-pyridin-2,4-dicarbonsäurediamid) ebenfalls mit aufgeführt. Überraschenderweise zeigen die erfindungsgemäßen Verbindungen sogar schon nach oraler Verabreichung eine bessere Wirksamkeit, als die i.p. verabreichte Verbindung aus Beispiel 2 der DE-A 37 03 959.

Tabelle 2

| Substanz aus Beispiel | Dosierung | Leber-Hydroxyprolin [% Hemmung] | 7s-(IV)-Serum-Kollagen [% Hemmung] | Verabreichung |
|---|---|---|---|---|
| 1 | 2x2 mg | 62 | 28 | p.o. |
| | 2x10 mg | 90 | 67 | p.o. |
| 2 | 2x2 mg | 25 | 2 | p.o. |
| | 2x10 mg | 60 | 35 | p.o. |
| 2 (aus DE-A 3703959) | 2x25 mg | 55 | 48 | i.p. |
| 3 (aus DE-A 3703959) | 2x25 mg | 49 | 11 | p.o. |

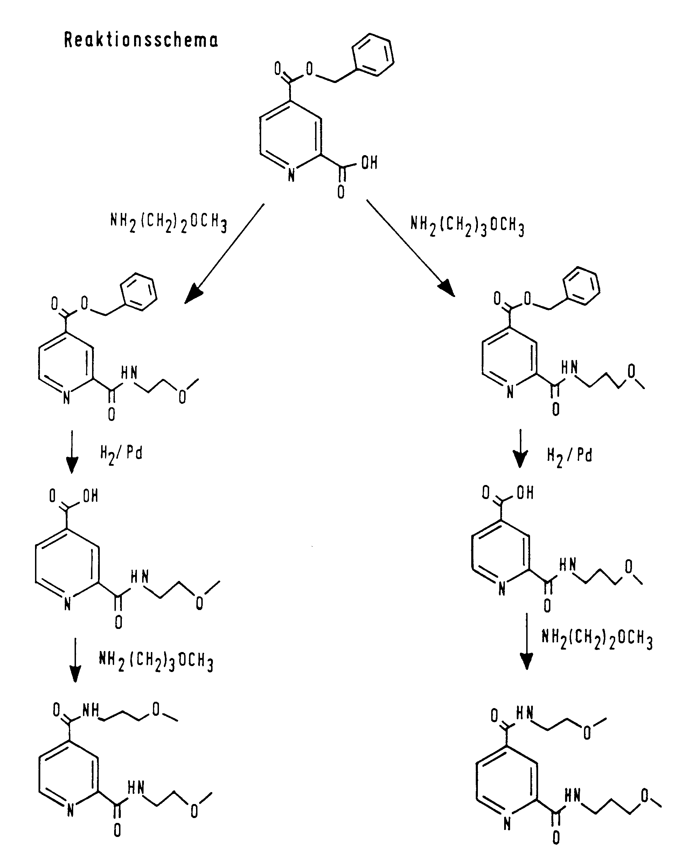

**Patentansprüche**

**Patentansprüche für folgende Vertragsstaaten : AT, BE, CH, DE, DK, FR, GB, IT, LI, LU, NL, SE**

**1.** N,N′-Bis(alkoxyalkyl)-pyridin-2,4-dicarbonsäurediamide der Formel I

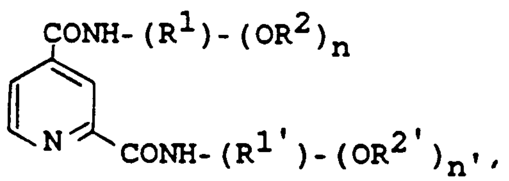

(I)

worin

| | |
|---|---|
| $R^1$ | lineares oder verzweigtes $C_1$-$C_4$-Alkandiyl, |
| $R^2$ | unverzweigtes $C_1$-$C_4$ Alkyl oder Wasserstoff und |
| n | 1 oder 2 bedeutet und |
| $R^{1'}$, $R^{2'}$ und n′ | die selben Bedeutungen wie $R^1$ $R^2$ und n haben, wobei $R^1$ und $R^{1'}$, $R^2$ und $R^{2'}$ und n und n′ identisch oder verschieden sind, |

sowie deren physiologisch verträgliche Salze, ausgenommen N,N′-Bis(2-methoxyethyl)-pyridin-2,4-dicarbonsäurediamid und N,N′-Bis(2-hydroxyethyl)-pyridin-2,4-dicarbonsäurediamid.

**2.** Verbindungen der Formel I nach Anspruch 1, dadurch gekennzeichnet, daß jeweils $R^1$ und $R^{1'}$, $R^2$ und $R^{2'}$ und n und n′ die gleiche Bedeutung haben.

**3.** Verbindungen der Formel I nach Anspruch 1, dadurch gekennzeichnet, daß die Substituenten $-(R^1)-(OR^2)n$ und $-(R^{1'})-(OR^{2'})n'$ unterschiedlich sind.

**4.** Verbindung der Formel I nach Anspruch 1, 2 oder 3, dadurch gekennzeichnet, daß
$R^1$ lineares oder verzweigtes $C_1$-$C_3$-Alkyl und
$R^2$ unverzweigtes $C_1$-$C_2$-Alkyl oder Wasserstoff bedeutet.

**5.** Verbindung der Formel

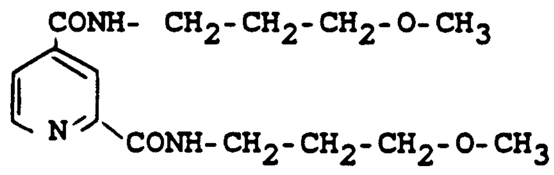

sowie ihre physiologisch verträglichen Salze.

**6.** Verbindung der Formel

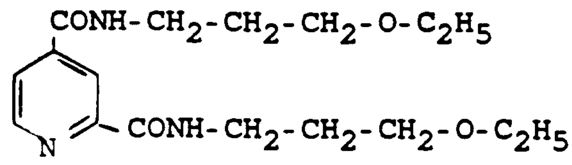

sowie ihre physiologisch verträglichen Salze.

**7.** Verbindung der Formel

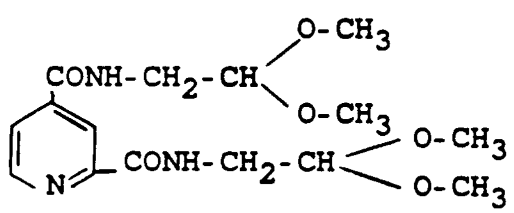

sowie ihre physiologisch verträglichen Salze.

**8.** Verbindung der Formel

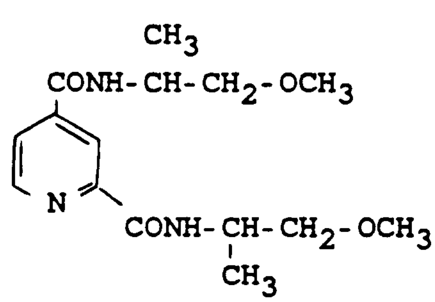

sowie ihre physiologisch verträglichen Salze.

**9.** Verbindung der Formel

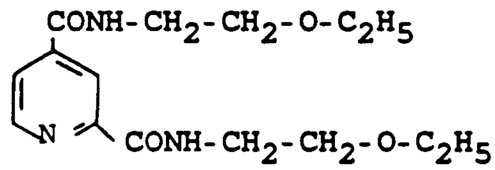

sowie ihre physiologisch verträglichen Salze.

**10.** Verbindung der Formel

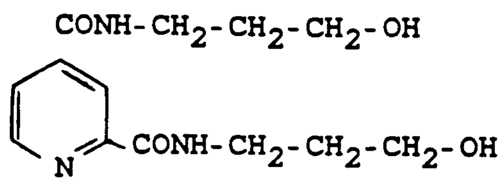

sowie ihre physiologisch verträglichen Salze.

**11.** Verbindung der Formel

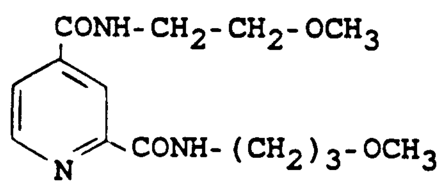

sowie ihre physiologisch verträglichen Salze.

**12.** Verbindung der Formel

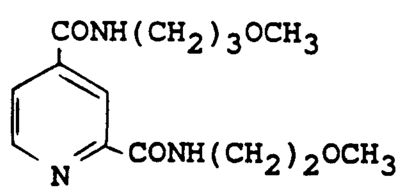

sowie ihre physiologisch verträglichen Salze.

**13.** Verfahren zur Herstellung von N,N'-Bis-(alkoxyalkyl)-pyridin-2,4-dicarbonsäurediamiden der Formel I

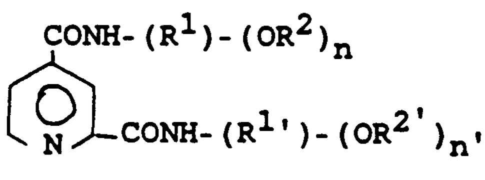

(I)

worin

| | |
|---|---|
| $R^1$ | lineares oder verzweigtes $C_1$-$C_4$-Alkandiyl, |
| $R^2$ | unverzweigtes $C_1$-$C_4$-Alkyl oder Wasserstoff |
| n | 1 oder 2 bedeutet und |
| $R^{1'}$, $R^{2'}$ und n' | die selben Bedeutungen wie $R^1$, $R^2$ und n haben, wobei $R^1$ und $R^{1'}$, $R^2$ und $R^{2'}$ und n und n' identisch oder verschieden sind, ausgenommen N,N'-Bis(2-methoxyethyl)-pyridin-2,4-dicarbonsäurediamid und N,N'-Bis(2-hydroxyethyl)-pyridin-2,4-dicarbonsäurediamid, |

dadurch gekennzeichnet, daß man Pyridin-2,4-dicarbonsäurehalogenid mit einem Alkoxyalkylamin oder Hydroxylalkylamin umsetzt.

**14.** Verfahren zur Herstellung von N,N'-Bis-(alkoxyalkyl)-pyridin-2,4-dicarbonsäurediamiden der Formel I

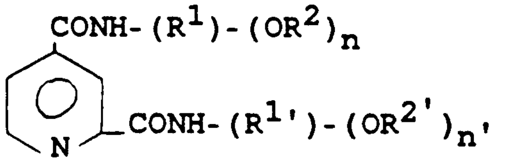

(I)

worin

| | |
|---|---|
| $R^1$ | lineares oder verzweigtes $C_1$-$C_4$-Alkandiyl, |
| $R^2$ | unverzweigtes $C_1$-$C_4$-Alkyl oder Wasserstoff |
| n | 1 oder 2 bedeutet und |
| $R^{1'}$, $R^{2'}$ und n' | dieselben Bedeutungen wie $R^1$, $R^2$ und n haben, wobei $R^1$ und $R^{1'}$, $R^2$ und $R^{2'}$ und n und n' identisch oder verschieden sind, |

dadurch gekennzeichnet, daß man

A)

1. Pyridin-2,4-dicarbonsäure mit mindestens zwei Äquivalenten eines Halogenierungsmittels versetzt, und daß man
2. mindestens 2 Äquivalente Hydroxyalkylamin oder Alkoxyalkylamin der Formel II oder II'

$H_2N$-$(R^1)$-$(OR^2)_n$ (II)

$H_2N$-$(R^{1'})$-$(OR^{2'})_{n'}$ (II')

worin

| | |
|---|---|
| $R^1$ und $R^{1'}$ | lineares oder verzweigtes $C_1$-$C_4$-Alkandiyl, |
| $R^2$ und $R^{2'}$ | unverzweigtes $C_1$-$C_4$-Alkyl oder Wasserstoff |

und

| | |
|---|---|
| n und n' | 1 oder 2 bedeutet und |
| $R^1$ und $R^{1'}$, $R^2$ und $R^{2'}$ | sowie n und n' identisch oder verschieden sind, wobei aber II und II' verschieden sind, |

in einem Lösungsmittel löst

und daß man dann die gemäß 1. hergestellte Lösung mit der gemäß 2. hergestellten Lösung zur Reaktion bringt, oder daß man

B) das so erhaltene N,N'-Bis(alkoxyalkyl)-pyridin-2,4-dicarbonsäurediamid in die Bis(hydroxyalkyl)-verbindung überführt oder das man

C) das gemäß A) 1. hergestellte Pyridin-2,4-dicarbonsäurehalogenid mit einem substituierten oder unsubstituierten Benzylalkohol zum Pyridin-2,4-dicarbonsäurebenzylester umsetzt, den Benzylester selektiv in 2-Position des Pyridins verseift, gemäß Verfahrensvariante A) 1. die freie Carbonsäure in 2-Position erneut in das Säurehalogenid überführt und die so erhaltene Verbindung mit einer gemäß A) 2. hergestellten Lösung unter Verwendung einer Verbindung der Formel II' versetzt, wobei ein Pyridin-4-carbonsäurebenzylestercarbonsäureamid entsteht, und daß man anschließend die Benzylschutzgruppe in 4-Position hydrogenolytisch abspaltet, die freie Carbonsäure gemäß Verfahrensvariante A) 1. erneut in das Säurechlorid überführt, welches dann mit einer gemäß A) 2. hergestellten

Lösung unter Verwendung einer Verbindung der Formel II versetzt wird, wobei eine unsymmetrisch substituierte Verbindung der Formel I entsteht und daß man ggf. anschließend die erhaltene Verbindung der Formel I in ihr physiologisch verträgliches Salz überführt.

**15.** Verbindungen nach einem der Ansprüche 1 bis 12 zur Inhibierung der Prolin- und Lysinhydroxylase.

**16.** Verbindungen nach einem der Ansprüche 1 bis 12 zur Anwendung als Fibrosuppressiva und Immunsuppresiva.

**17.** Arzneimittel, enthaltend eine Verbindung der Formel I nach einem der Ansprüche 1-12 und/oder eines ihrer physiologisch verträglichen Salze mit verträglichen pharmazeutischen Trägern.

**Patentansprüche für folgende Vertragsstaaten : ES, GR**

**1.** Verfahren zur Herstellung von N,N'-Bis-(alkoxyalkyl)-pyridin-2,4-dicarbonsäurediamiden der Formel I

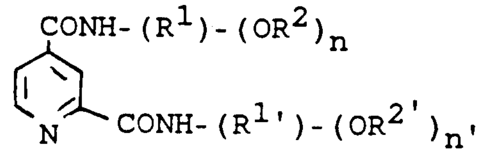

(I)

worin

| | |
|---|---|
| $R^1$ | lineares oder verzweigtes $C_1$-$C_4$-Alkandiyl, |
| $R^2$ | unverzweigtes $C_1$-$C_4$-Alkyl oder Wasserstoff |
| n | 1 oder 2 bedeutet und |
| $R^{1'}$, $R^{2'}$ und n' | die selben Bedeutungen wie $R^1$, $R^2$ und n haben, wobei $R^1$ und $R^{1'}$, $R^2$ und $R^{2'}$ und n und n' identisch oder verschieden sind, ausgenommen N,N'-Bis(2-methoxyethyl)-pyridin-2,4-dicarbonsäurediamid und N,N'-Bis(2-hydroxyethyl)-pyridin-2,4-dicarbonsäurediamid, |

dadurch gekennzeichnet, daß man Pyridin-2,4 dicarbonsäurehalogenid mit einem Alkoxyalkylamin oder Hydroxylalkylamin umsetzt.

**2.** Verfahren zur Herstellung von N,N'-Bis-(alkoxyalkyl)-pyridin-2,4-dicarbonsäurediamiden der Formel I

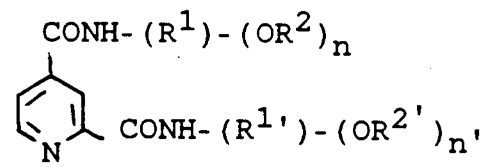

(I)

worin

| | |
|---|---|
| $R^1$ | lineares oder verzweigtes $C_1$-$C_4$-Alkandiyl, |
| $R^2$ | unverzweigtes $C_1$-$C_4$-Alkyl oder Wasserstoff |
| n | 1 oder 2 bedeutet und |
| $R^{1'}$, $R^{2'}$ und n' | dieselben Bedeutungen wie $R^1$, $R^2$ und n haben, wobei $R^1$ und $R^{1'}$, $R^2$ und $R^{2'}$ und n und n' identisch oder verschieden sind, |

dadurch gekennzeichnet, daß man

A)

1. Pyridin-2,4-dicarbonsäure mit mindestens zwei Äquivalenten eines Halogenierungsmittels versetzt, und daß man
2. mindestens 2 Äquivalente Hydroxyalkylamin oder Alkoxyalkylamin der Formel II oder II'

$H_2N$-$(R^1)$-$(OR^2)_n$ (II)

$H_2N-(R^{1'})-(OR^{2'})_{n'}$ (II')

worin

$R^1$ und $R^{1'}$ , lineares oder verzweigtes $C_1$-$C_4$-Alkandiyl,

$R^2$ und $R^{2'}$ unverzweigtes $C_1$-$C_4$-Alkyl oder Wasserstoff

und

n und n' 1 oder 2 bedeutet und

$R^1$ und $R^{1'}$ $R^2$ und $R^{2'}$ sowie n und n' identisch oder verschieden sind, wobei aber II und II' verschieden sind,

in einem Lösungsmittel löst

und daß man dann die gemäß 1. hergestellte Lösung mit der gemäß 2. hergestellten Lösung zur Reaktion bringt, oder daß man

B) das so erhaltene N,N'-Bis(alkoxyalkyl)-pyridin-2,4-dicarbonsäurediamid in die Bis(hydroxyalkyl)-verbindung überführt oder das man

C) das gemäß A) 1. hergestellte Pyridin-2,4-dicarbonsäurehalogenid mit einem substituierten oder unsubstituierten Benzylalkohol zum Pyridin-2,4-dicarbonsäurebenzylester umsetzt, den Benzylester selektiv in 2-position des Pyridins verseift, gemäß Verfahrensvariante A) 1. die freie Carbonsäure in 2-position erneut in das Säurehalogenid überführt und die so erhaltene Verbindung mit einer gemäß A) 2. hergestellten Lösung unter Verwendung einer Verbindung der Formel II' versetzt, wobei ein Pyridin-4-carbonsäurebenzylestercarbonsäureamid entsteht, und daß man anschließend die Benzylschutzgruppe in 4-position hydrogenolytisch abspaltet, die freie Carbonsäure gemäß Verfahrensvariante A) 1. erneut in das Säurechlorid überführt, welches dann mit einer gemäß A) 2. hergestellten Lösung unter Verwendung einer Verbindung der Formel II versetzt wird, wobei eine unsymmetrisch substituierte Verbindung der Formel I entsteht und daß man ggf. anschließend die erhaltene Verbindung der Formel I in ihr physiologisch verträgliches Salz überführt.

**3.** Verfahren nach Anspruch 1 oder 2, dadurch gekennzeichnet, daß in Formel I jeweils $R^1$ und $R^{1'}$, $R^2$ und $R^{2'}$ und n und n' die gleiche Bedeutung haben.

**4.** Verfahren nach Anspruch 1 oder 2, dadurch gekennzeichnet, daß in Formel I die Substituenten $-(R^1)-(OR^2)n$ und $-(R^{1'})-(OR^{2'})n'$ unterschiedlich sind.

**5.** Verfahren nach Anspruch 1 oder 2, dadurch gekennzeichnet, daß $R^1$ lineares oder verzweigtes $C_1$-$C_3$-Alkyl und $R^2$ unverzweigtes $C_1$-$C_2$-Alkyl oder Wasserstoff bedeutet.

**6.** Verfahren nach Anspruch 1 oder 2, dadurch gekennzeichnet, daß eine Verbindung der Formel

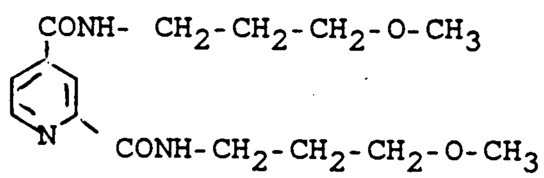

sowie ihre physiologisch verträglichen Salze hergestellt werden.

**7.** Verfahren nach Anspruch 1 oder 2, dadurch gekennzeichnet, daß eine Verbindung der Formel

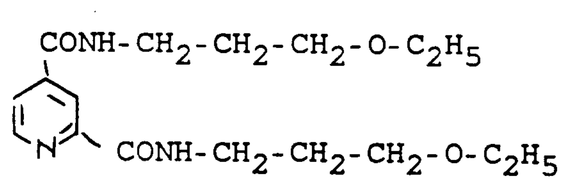

sowie ihre physiologisch verträglichen Salze hergestellt werden.

8. Verfahren nach Anspruch 1 oder 2, dadurch gekennzeichnet daß eine Verbindung der Formel

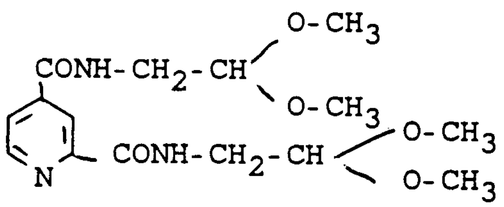

sowie ihre physiologisch verträglichen Salze hergestellt werden.

9. Verfahren nach Anspruch 1 oder 2, dadurch gekennzeichnet, daß eine Verbindung der Formel

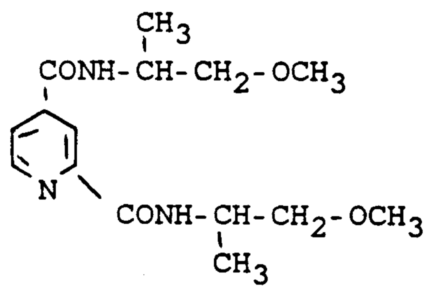

sowie ihre physiologisch verträglichen Salze hergestellt werden.

10. Verfahren nach Anspruch 1 oder 2, dadurch gekennzeichnet, daß eine Verbindung der Formel

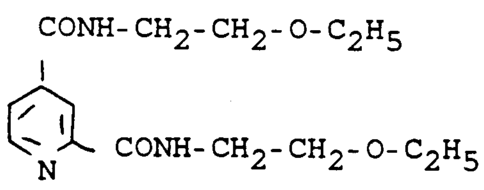

sowie ihre physiologisch verträglichen Salze hergestellt werden.

11. Verfahren nach Anspruch 1 oder 2, dadurch gekennzeichnet, daß eine Verbindung der Formel

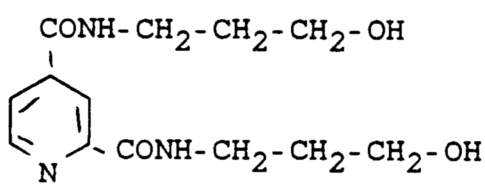

sowie ihre physiologisch verträglichen Salze hergestellt werden.

**12.** Verfahren nach Anspruch 1 oder 2, dadurch gekennzeichnet, daß eine Verbindung der Formel

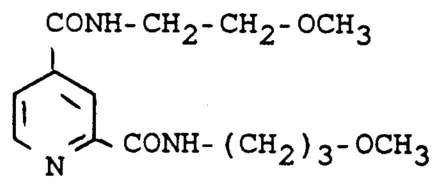

sowie ihre physiologisch verträglichen Salze hergestellt werden.

**13.** Verfahren nach Anspruch 1 oder 2, dadurch gekennzeichnet, daß eine Verbindung der Formel

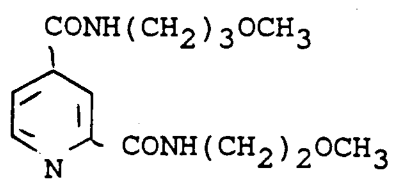

sowie ihre physiologisch verträglichen Salze hergestellt werden.

**Claims**

**Claims for the following Contracting States : AT, BE, CH, DE, DK, FR, GB, IT, LI, LU, NL, SE**

**1.** An N,N'-bis(alkoxyalkyl)pyridine-2,4-dicarboxylic acid diamide of the formula I

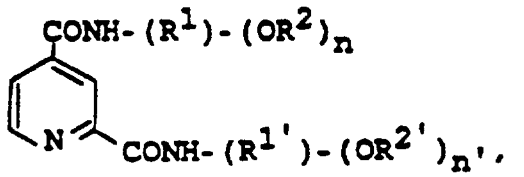

(I)

in which

| | |
|---|---|
| $R^1$ | denotes linear or branched $C_1$-$C_4$-alkanediyl, |
| $R^2$ | denotes unbranched $C_1$-$C_4$-alkyl or hydrogen, |
| n | denotes 1 or 2 and |
| $R^{1'}$, $R^{2'}$ and n' | have the same meanings as $R^1$, $R^2$ and n, $R^1$ and $R^{1'}$, and $R^2$ and $R^{2'}$ and n and n' being identical or different, |

or a physiologically tolerated salt thereof, excluding N,N'-bis(2-methoxyethyl)pyridine-2,4-dicarboxylic acid diamide and N,N'-bis(2-hydroxyethyl)pyridine-2,4-dicarboxylic acid diamide.

**2.** A compound of the formula I as claimed in claim 1, wherein in each case $R^1$ and $R^{1'}$, $R^2$ and $R^{2'}$ and n and n' have the same meaning.

**3.** A compound of the formula I as claimed in claim 1, wherein the substituents $-(R^1)-(OR^2)_n$ and $-(R^{1'})-(OR^{2'})_{n'}$ are different.

**4.** A compound of the formula I as claimed in claim 1, 2 or 3, wherein

$R^1$ denotes linear or branched $C_1$-$C_3$-alkyl and

$R^2$ denotes unbranched $C_1$-$C_2$-alkyl or hydrogen.

**5.** The compound of the formula

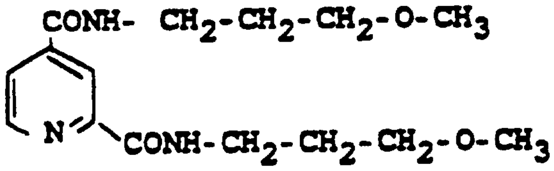

or one of its physiologically tolerated salts.

**6.** The compound of the formula

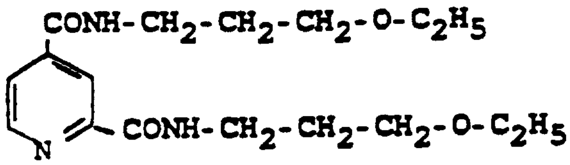

or one of its physiologically tolerated salts.

**7.** The compound of the formula

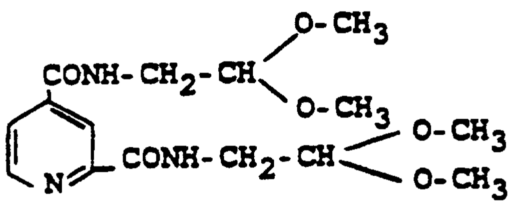

or one of its physiologically tolerated salts.

**8.** The compound of the formula

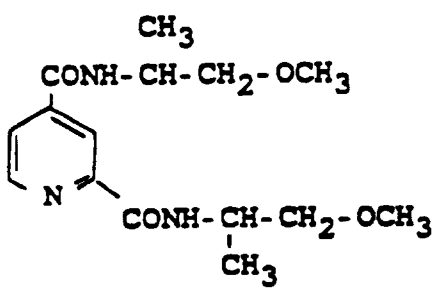

or one of its physiologically tolerated salts.

**9.** The compound of the formula

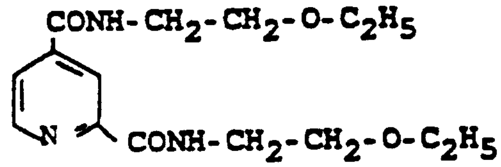

or one of its physiologically tolerated salts.

**10.** The compound of the formula

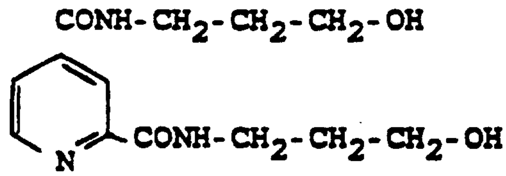

or one of its physiologically tolerated salts.

**11.** The compound of the formula

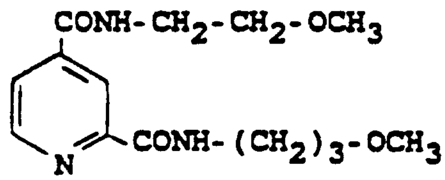

or one of its physiologically tolerated salts.

**12.** The compound of the formula

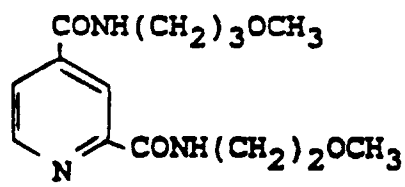

or one of its physiologically tolerated salts.

**13.** A process for the preparation of an N,N'-bis(alkoxyalkyl)pyridine-2,4-dicarboxylic acid diamide of the formula I

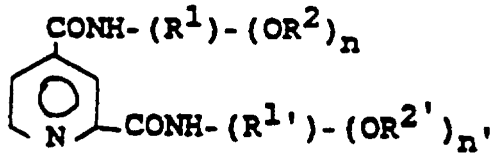

(I)

in which

| | |
|---|---|
| $R^1$ | denotes linear or branched $C_1$-$C_4$-alkanediyl, |
| $R^2$ | denotes unbranched $C_1$-$C_4$-alkyl or hydrogen, |
| n | denotes 1 or 2 and |
| $R^{1'}$, $R^{2'}$ and n' | have the same meanings as $R^1$, $R^2$ and n, $R^1$ and $R^{1'}$, and $R^2$ and $R^{2'}$ and n and n' being identical or different, excluding N,N'-bis(2-methoxyethyl)pyridine-2,4-dicarboxylic acid diamide and N,N'-bis(2-hydroxyethyl)pyridine-2,4-dicarboxylic acid diamide, |

which comprises reacting a pyridine-2,4-dicarboxylic acid halide with an alkoxyalkylamine or hydroxyalkylamine.

**14.** A process for the preparation of an N,N'-bis(alkoxyalkyl)pyridine-2,4-dicarboxylic acid diamide of the formula I

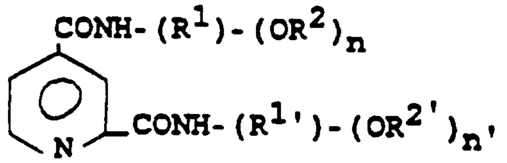

(I)

in which

| | |
|---|---|
| $R^1$ | denotes linear or branched $C_1$-$C_4$-alkanediyl, |
| $R^2$ | denotes unbranched $C_1$-$C_4$-alkyl or hydrogen, |
| n | denotes 1 or 2 and |
| $R^{1'}$, $R^{2'}$ and n' | have the same meanings as $R^1$, $R^2$ and n, $R^1$ and $R^{1'}$, and $R^2$ and $R^{2'}$ and n and n' being identical or different, |

which comprises

A)

1. adding at least two equivalents of a halogenating agent to pyridine-2,4-dicarboxylic acid and
2. dissolving at least 2 equivalents of a hydroxyalkylamine or alkoxyalkylamine of the formula II or II'

$H_2N$-($R^1$)-($OR^2$)$_n$ (II)

$H_2N$-($R^{1'}$)-($OR^{2'}$)$_{n'}$ (II')

in which

| | |
|---|---|
| $R^1$ and $R^{1'}$ | denote linear or branched $C_1$-$C_4$-alkanediyl, |
| $R^2$ and $R^{2'}$ | denote unbranched $C_1$-$C_4$-alkyl or hydrogen, |
| n and n' | denote 1 or 2 and |
| $R^1$ and $R^{1'}$, $R^2$ and $R^{2'}$ and n and n' | are identical or different, but II and II' are different, |

in a solvent

and then reacting the solution prepared according to 1. with the solution prepared according to 2., or

B) converting the resulting N,N'-bis(alkoxyalkyl)pyridine-2,4-dicarboxylic acid diamide into the bis-(hydroxyalkyl) compound, or

C) reacting the pyridine-2,4-dicarboxylic acid halide prepared according to A) 1. with a substituted or unsubstituted benzyl alcohol to give the benzyl pyridine-2,4-dicarboxylate, hydrolyzing the benzyl

ester selectively in the 2-position of the pyridine, converting the free carboxylic acid in the 2-position into the acid halide again in accordance with process variant A) 1. and adding a solution prepared according to A) 2. using a compound of the formula II' to the compound thus obtained, a pyridine-4-(carboxylic acid benzyl ester)-carboxylic acid amide being formed, and then eliminating the benzyl protective group in the 4-position by hydrogenolysis, converting the free carboxylic acid into the acid chloride again in accordance with process variant A) 1., and subsequently adding a solution prepared according to A) 2. using a compound of the formula II, an unsymmetrically substituted compound of the formula I being formed, and if appropriate then converting the resulting compound of the formula I into its physiologically tolerated salt.

**15.** A compound as claimed in any one of claims 1 to 12 for inhibition of proline hydroxylase and lysine hydroxylase.

**16.** A compound as claimed in any one of claims 1 to 12 for use as a fibrosuppressant and immunosuppressant.

**17.** A pharmaceutical containing a compound of the formula I as claimed in any one of claims 1-12 and/or one of its physiologically tolerated salts with tolerated pharmaceutical excipients.

**Claims for the following Contracting States : ES, GR**

**1.** A process for the preparation of an N,N'-bis(alkoxyalkyl)pyridine-2,4-dicarboxylic acid diamide of the formula I

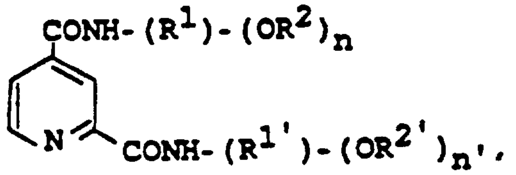

(I)

in which

| | |
|---|---|
| $R^1$ | denotes linear or branched $C_1$-$C_4$-alkanediyl, |
| $R^2$ | denotes unbranched $C_1$-$C_4$-alkyl or hydrogen, |
| n | denotes 1 or 2 and |
| $R^{1'}$, $R^{2'}$ and n' | have the same meanings as $R^1$, $R^2$ and n, $R^1$ and $R^{1'}$, and $R^2$ and $R^{2'}$ and n and n' being identical or different, |

or a physiologically tolerated salt thereof, excluding N,N'-bis(2-methoxyethyl)pyridine-2,4-dicarboxylic acid diamide and N,N'-bis(2-hydroxyethyl)pyridine-2,4-dicarboxylic acid diamide, which comprises reacting a pyridine-2,4-dicarboxylic acid halide with an alkoxyalkylamine or hydroxyalkylamine.

**2.** A process for the preparation of an N,N'-bis(alkoxyalkyl)pyridine-2,4-dicarboxylic acid diamide of the formula I

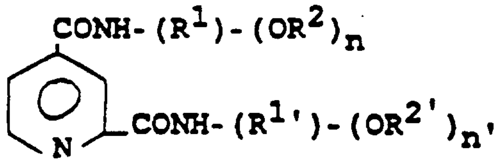

(I)

in which

| | |
|---|---|
| $R^1$ | denotes linear or branched $C_1$-$C_4$-alkanediyl, |
| $R^2$ | denotes unbranched $C_1$-$C_4$-alkyl or hydrogen, |
| n | denotes 1 or 2 and |
| $R^{1'}$, $R^{2'}$ and n' | have the same meanings as $R^1$, $R^2$ and n, $R^1$ and $R^{1'}$, and $R^2$ and $R^{2'}$ and n and n' |

being identical or different,

which comprises

A)

1. adding at least two equivalents of a halogenating agent to pyridine-2,4-dicarboxylic acid and
2. dissolving at least 2 equivalents of a hydroxyalkylamine or alkoxyalkylamine of the formula II or II'

$H_2N-(R^1)-(OR^2)_n$ (II)

$H_2N-(R^{1'})-(OR^{2'})_{n'}$ (II')

in which

| | |
|---|---|
| $R^1$ and $R^{1'}$ | denote linear or branched $C_1$-$C_4$-alkanediyl, |
| $R^2$ and $R^{2'}$ | denote unbranched $C_1$-$C_4$-alkyl or hydrogen, |
| n and n' | denote 1 or 2 and |
| $R^1$ and $R^{1'}$, $R^2$ and $R^{2'}$ and n and n' | are identical or different, but II and II' are different, |

in a solvent

and then reacting the solution prepared according to 1. with the solution prepared according to 2., or

B) converting the resulting N,N'-bis(alkoxyalkyl)pyridine-2,4-dicarboxylic acid diamide into the bis-(hydroxyalkyl) compound, or

C) reacting the pyridine-2,4-dicarboxylic acid halide prepared according to A) 1. with a substituted or unsubstituted benzyl alcohol to give the benzyl pyridine-2,4-dicarboxylate, hydrolyzing the benzyl ester selectively in the 2-position of the pyridine, converting the free carboxylic acid in the 2-position into the acid halide again in accordance with process variant A) 1. and adding a solution prepared according to A) 2. using a compound of the formula II' to the compound thus obtained, a pyridine-4-(carboxylic acid benzyl ester)-carboxylic acid amide being formed, and then eliminating the benzyl protective group in the 4-position by hydrogenolysis, converting the free carboxylic acid into the acid chloride again in accordance with process variant A) 1., and subsequently adding a solution prepared according to A) 2. using a compound of the formula II, an unsymmetrically substituted compound of the formula I being formed, and if appropriate then converting the resulting compound of the formula I into its physiologically tolerated salt.

**3.** The process as claimed in claim 1 or 2, wherein in formula I in each case $R^1$ and $R^{1'}$, $R^2$ and $R^{2'}$ and n and n' have the same meaning.

**4.** The process as claimed in claim 1 or 2, wherein in formula I the substituents $-(R^1)-(OR^2)_n$ and $-(R^{1'})-(OR^{2'})_{n'}$ are different.

**5.** The process as claimed in claim 1 or 2, wherein $R^1$ denotes linear or branched $C_1$-$C_3$-alkyl and $R^2$ denotes unbranched $C_1$-$C_2$-alkyl or hydrogen.

**6.** The process as claimed in claim 1 or 2, which comprises preparing the compound of the formula

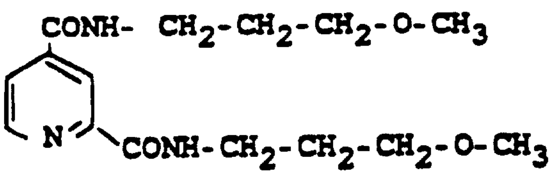

or one of its physiologically tolerated salts.

7. The process as claimed in claim 1 or 2, which comprises preparing the compound of the formula

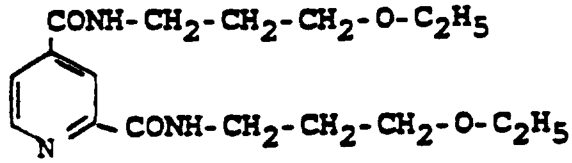

or one of its physiologically tolerated salts.

8. The process as claimed in claim 1 or 2, which comprises preparing the compound of the formula

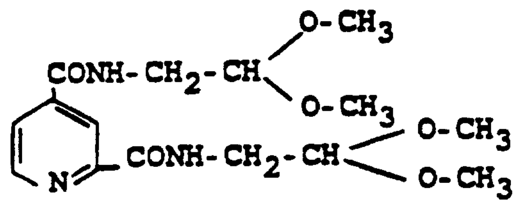

or one of its physiologically tolerated salts.

9. The process as claimed in claim 1 or 2, which comprises preparing the compound of the formula

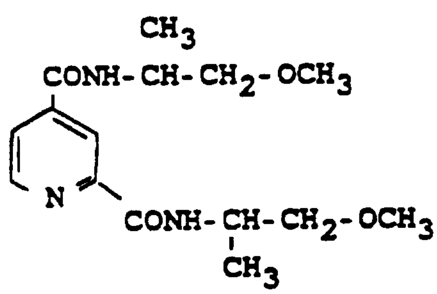

or one of its physiologically tolerated salts.

10. The process as claimed in claim 1 or 2, which comprises preparing the compound of the formula

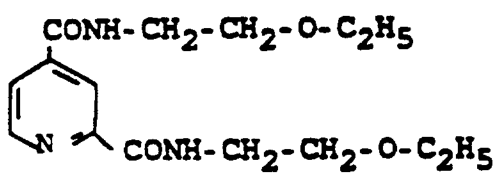

or one of its physiologically tolerated salts.

**11.** The process as claimed in claim 1 or 2, which comprises preparing the compound of the formula

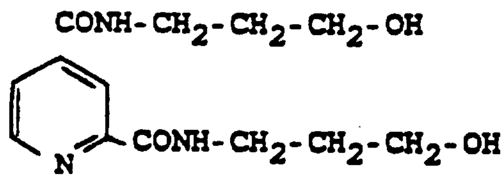

or one of its physiologically tolerated salts.

**12.** The process as claimed in claim 1 or 2, which comprises preparing the compound of the formula

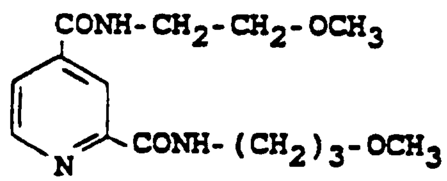

or one of its physiologically tolerated salts.

**13.** The process as claimed in claim 1 or 2, which comprises preparing the compound of the formula

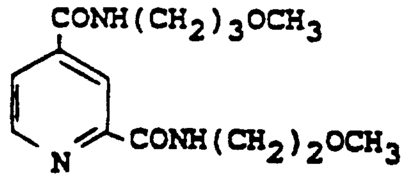

or one of its physiologically tolerated salts.

**Revendications**

**Revendications pour les Etats contractants suivants : AT, BE, CH, DE, DK, FR, GB, IT, LI, LU, NL, SE**

**1.** N,N'-bis(alcoxyalkyl)-pyridine-2,4-dicarboxamides de formule I,

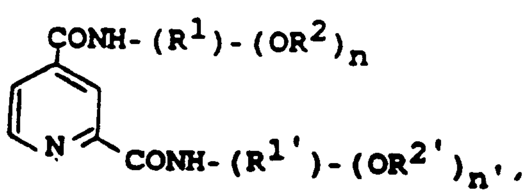

(I)

dans laquelle
$R^1$ représente un groupe alcanediyle en $C_{1-4}$, linéaire ou ramifié,
$R^2$ représente un groupe alkyle en $C_{1-4}$ non ramifié ou un atome d'hydrogène,
n vaut 1 ou 2, et
$R^{1'}$, $R^{2'}$ et n' ont les mêmes définitions que $R^1$, $R^2$ et n, avec la possibilité que $R^1$ et $R^{1'}$, $R^2$ et $R^{2'}$ ainsi que n et n' puissent être identiques ou différents,
ainsi que leurs sels physiologiquement compatibles, à l'exception du N,N'-bis(2-méthoxyéthyl)-pyridine-2,4-dicarboxamide et du N,N'-bis(2-hydroxyéthyl)-pyridine-2,4-dicarboxamide.

**2.** Composés de formule I selon la revendication 1, caractérisés en ce que $R^1$ et $R^{1'}$, $R^2$ et $R^{2'}$ ainsi que n et n' sont respectivement identiques.

**3.** Composés de formule I selon la revendication 1, caractérisés en ce que les substituants $-(R^1)-(OR^2)n$ et $-(R^{1'})-(OR^{2'})n'$ sont différents.

**4.** Composé de formule I selon la revendication 1, 2 ou 3, caractérisé en ce que
$R^1$ représente un groupe alkyle en $C_{1-3}$, linéaire ou ramifié, et
$R^2$ représente un groupe alkyle en $C_{1-2}$ non ramifié ou un atome d'hydrogène.

**5.** Composé de formule

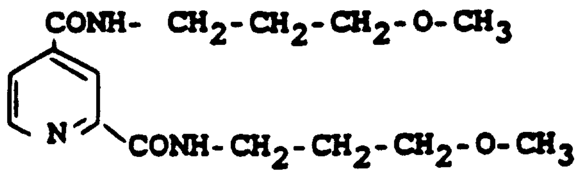

ainsi que ses sels physiologiquement compatibles

**6.** Composé de formule

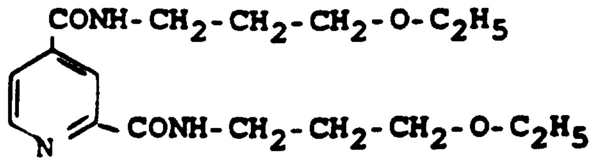

ainsi que ses sels physiologiquement compatibles

**7.** Composé de formule

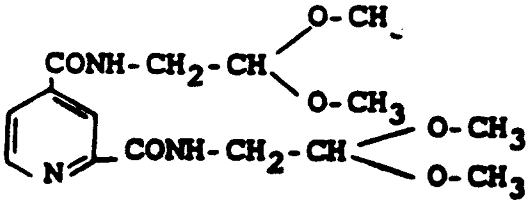

ainsi que ses sels physiologiquement compatibles

**8.** Composé de formule

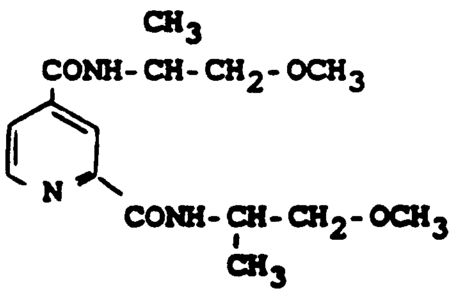

ainsi que ses sels physiologiquement compatibles

**9.** Composé de formule

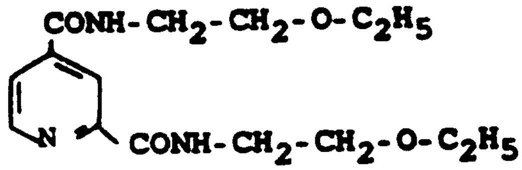

ainsi que ses sels physiologiquement compatibles

**10.** Composé de formule

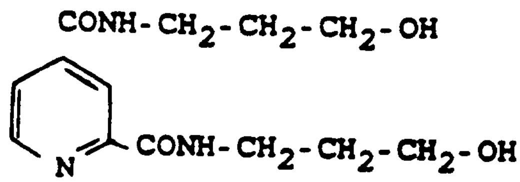

ainsi que ses sel physiologiquement compatibles

**11.** Composé de formule

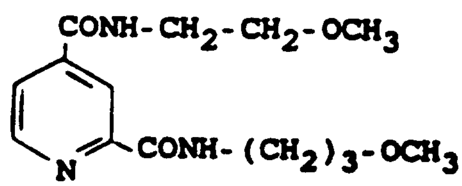

ainsi que ses sels physiologiquement compatibles

**12.** Composé de formule

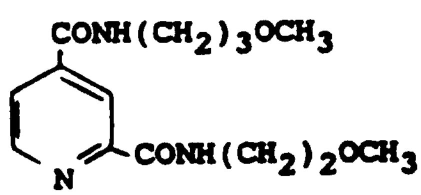

ainsi que ses sels physiologiquement compatibles.

**13.** Procédé de préparation de N,N'-bis(alcoxyalkyl)-pyridine-2,4-dicarboxamides de formule I,

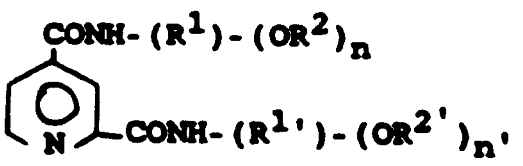

(I)

dans laquelle

$R^1$ représente un groupe alcanediyle en $C_{1-4}$, linéaire ou ramifié,
$R^2$ représente un groupe alkyle en $C_{1-4}$ non ramifié ou un atome d'hydrogène,
n vaut 1 ou 2, et
$R^{1'}$, $R^{2'}$ et n' ont les mêmes définitions que $R^1$, $R^2$ et n, avec la possibilité que $R^1$ et $R^{1'}$, $R^2$ et $R^{2'}$ ainsi que n et n' puissent être identiques ou différents,
à l'exception du N,N'-bis(2-méthoxyéthyl)-pyridine-2,4-dicarboxamide et du N,N'-bis(2-hydroxyéthyl)-pyridine-2,4-dicarboxamide,
procédé caractérisé en ce qu'on fait réagir un halogénure d'acide pyridine-2,4-dicarboxylique avec une alcoxyalkylamine ou avec une hydroxyalkylamine.

**14.** Procédé de préparation de N,N'-bis(alcoxyalkyl)-pyridine-2,4-dicarboxamides de formule I,

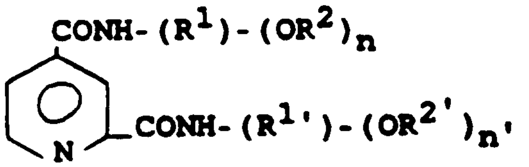

(I)

dans laquelle
$R^1$ représente un groupe alcanediyle en $C_{1-4}$, linéaire ou ramifié,
$R^2$ représente un groupe alkyle en $C_{1-4}$ non ramifié ou un atome d'hydrogène,
n vaut 1 ou 2, et
$R^{1'}$, $R^{2'}$ et n' ont les mêmes définitions que $R^1$, $R^2$ et n, avec la possibilité que $R^1$ et $R^{1'}$, $R^2$ et $R^{2'}$ ainsi que n et n' puissent être identiques ou différents,
caractérisé en ce que

A)

1. on mélange d'abord de l'acide pyridine-2,4-dicarboxylique avec au moins 2 équivalents d'un agent d'halogénation,
2. on dissout dans un solvant au moins 2 équivalents d'hydroxyalkylamine ou d'alcoxyalkylamine de formule II ou II'

$H_2N$-$(R^1)$-$(OR^2)_n$ (II)

$H_2N$-$(R^{1'})$-$(OR^{2'})_{n'}$ (II'),

dans lesquelles
$R^1$ et $R^{1'}$ représentent un groupe alcanediyle en $C_{1-4}$, linéaire ou ramifié,
$R^2$ et $R^{2'}$ représentent un groupe alkyle en $C_{1-4}$ non ramifié ou un atome d'hydrogène,
n et n' valent 1 ou 2, et
$R^1$ et $R^{1'}$, $R^2$ et $R^{2'}$ ainsi que n et n' sont identiques ou différents, II et II' étant toutefois différents,
et on fait réagir la solution préparée selon 1. avec la solution préparée selon 2., ou bien

B) on transforme le N,N'-bis(alcoxyalkyl)-pyridine-2,4-dicarboxamide ainsi obtenu en dérivé de bis-(hydroxyalkyle), ou

C) on fait réagir l'halogénure d'acide pyridine-2,4-dicarboxylique préparé selon A) 1. avec un alcool benzylique substitué ou non substitué, pour obtenir le pyridine-2,4-dicarboxylate de dibenzyle, on saponifie sélectivement, en position 2 de la pyridine, l'ester benzylique, on transforme à nouveau en halogénure d'aide l'acide carboxylique libre en position 2 selon la variante de procédé A) 1. et on fait réagir le composé ainsi obtenu avec une solution préparée selon A) 2., en utilisant un composé de formule II', ce qui produit un 2-amidopyridine-4-carboxylate de benzyle, puis on chasse par hydrogénolyse le groupe protecteur benzyle en position 4, on transforme à nouveau l'acide carboxylique libre, selon la variante de procédé A) 1., en chlorure d'acide que l'on fait alors réagir avec une solution préparée selon A) 2., en utilisant un composé de formule II, ce qui conduit a la formation d'un composé asymétriquement substitué de formule I, et on transforme éventuellement le composé de formule I obtenu en son sel physiologiquement compatible.

**15.** Composés selon une quelconque des revendications 1 à 12 pour l'inhibition des proline et lysine hydroxylases.

**16.** Composés selon une quelconque des revendications 1 à 12 pour leur application comme fibrosuppresseurs et immunosuppresseurs.

**17.** Médicaments, qui contiennent un composé de formule I selon une quelconque des revendications 1 à 12 et/ou un de leurs sels physiologiquement compatibles, conjointement avec des véhicules pharmaceutiquement acceptables.

**Revendications pour les Etats contractants suivants : ES, GR**

**1.** Procédé de préparation de N,N'-bis(alcoxyalkyl)-pyridine-2,4-dicarboxamides de formule I,

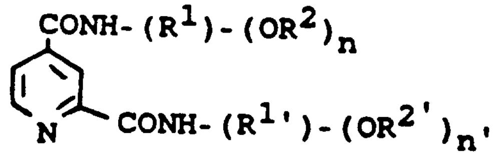

(I)

dans laquelle
$R^1$ représente un groupe alcanediyle en $C_{1-4}$, linéaire ou ramifié,
$R^2$ représente un groupe alkyle en $C_{1-4}$ non ramifié ou un atome d'hydrogène,
n vaut 1 ou 2, et
$R^{1'}$, $R^{2'}$ et n' ont les mêmes définitions que $R^1$, $R^2$ et n, avec la possibilité que $R^1$ et $R^{1'}$, $R^2$ et $R^{2'}$ ainsi que n et n' puissent être identiques ou différents,
à l'exception du N,N'-bis(2-méthoxyéthyl)-pyridine-2,4-dicarboxamide et du N,N'-bis(2-hydroxyéthyl)-pyridine-2,4-dicarboxamide,
procédé caractérisé en ce qu'on fait réagir un halogénure d'acide pyridine-2,4-dicarboxylique avec une alcoxyalkylamine ou avec une hydroxyalkylamine.

**2.** Procédé de préparation de N,N'-bis(alcoxyalkyl)-pyridine-2,4-dicarboxamides de formule I,

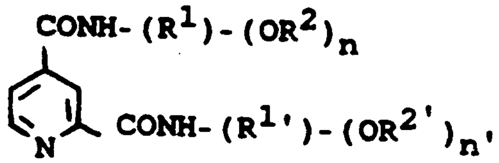

(I)

dans laquelle
$R^1$ représente un groupe alcanediyle en $C_{1-4}$, linéaire ou ramifié,
$R^2$ représente un groupe alkyle en $C_{1-4}$ non ramifié ou un atome d'hydrogène,
n vaut 1 ou 2, et
$R^{1'}$, $R^{2'}$ et n' ont les mêmes définitions que $R^1$, $R^2$ et n, avec la possibilité que $R^1$ et $R^{1'}$, $R^2$ et $R^{2'}$ ainsi que n et n' puissent être identiques ou différents,
caractérisé en ce que

A)

1. on mélange d'abord de l'acide pyridine-2,4-dicarboxylique avec au moins 2 équivalents d'un agent d'halogénation,
2. on dissout dans un solvant au moins 2 équivalents d'hydroxyalkylamine ou d'alcoxyalkylamine de formule II ou II'

$H_2N-(R^1)-(OR^2)_n$ (II)

$H_2N-(R^{1'})-(OR^{2'})_{n'}$ (II'),

dans lesquelles
$R^1$ et $R^{1'}$ représentent un groupe alcanediyle en $C_{1-4}$, linéaire ou ramifié,
$R^2$ et $R^{2'}$ représentent un groupe alkyle en $C_{1-4}$ non ramifié ou un atome d'hydrogène,
n et n' valent 1 ou 2, et
$R^1$ et $R^{1'}$, $R^2$ et $R^{2'}$ ainsi que n et n' sont identiques ou différents, II et II' étant toutefois différents,
et on fait réagir la solution préparée selon 1. avec la solution préparée selon 2., ou bien

B) on transforme le N,N'-bis(alcoxyalkyl)-pyridine-2,4-dicarboxamide ainsi obtenu en dérivé de bis-(hydroxyalkyle), ou

C) on fait réagir l'halogénure d'acide pyridine-2,4-dicarboxylique préparé selon A) 1. avec un alcool benzylique substitué ou non substitué, pour obtenir le pyridine-2,4-dicarboxylate de dibenzyle, on saponifie sélectivement, en position 2 de la pyridine, l'ester benzylique, on transforme à nouveau en halogénure d'acide l'acide carboxylique libre en position 2 selon la variante de procédé A) 1.et on fait réagir le composé ainsi obtenu avec une solution préparée selon A) 2., en utilisant un composé de formule II', ce qui produit un 2-amidopyridine-4-carboxylate de benzyle, puis on chasse par hydrogénolyse le groupe protecteur benzyle en position 4, on transforme à nouveau l'acide carboxylique libre, selon la variante de procédé A) 1., en chlorure d'acide que l'on fait alors réagir avec une solution préparée selon A) 2., en utilisant un composé de formule II, ce que conduit à la formation d'un composé asymétriquement substitué de formule I, et on transforme éventuellement le composé de formule I obtenu en son sel physiologiquement compatible.

**3.** Procédé selon la revendication 1 ou 2, caractérisé en ce que, dans la formule I, $R^1$ et $R^{1'}$, $R^2$ et $R^{2'}$ ainsi que n et n' sont respectivement identiques.

**4.** Procédé selon la revendication 1 ou 2, caractérisé en ce que, dans la formule I, les substituants $-(R^1)-(OR^2)n$ et $-(R^{1'})-(OR^{2'})n'$ sont différents.

**5.** Procédé selon la revendication 1 ou 2, caractérisé en ce que
$R^1$ représente un groupe alkyle en $C_{1-3}$, linéaire ou ramifié, et
$R^2$ représente un groupe alkyle en $C_{1-2}$ non ramifié ou un atome d'hydrogène.

**6.** Procédé selon la revendication 1 ou 2, caractérisé en ce qu'on prépare un composé de formule

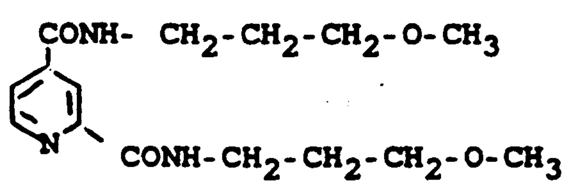

ainsi que ses sels physiologiquement compatibles

**7.** Procédé selon la revendication 1 ou 2, caractérisé en ce qu'on prépare un composé de formule

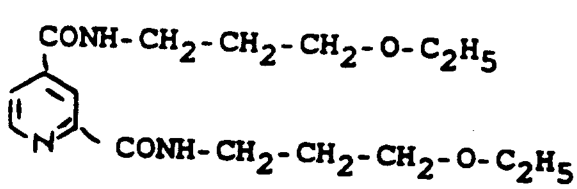

ainsi que ses sels physiologiquement compatibles

**8.** Procédé selon la revendication 1 ou 2, caractérisé en ce qu'on prépare un composé de formule

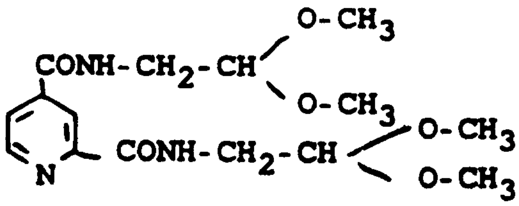

ainsi que ses sels physiologiquement compatibles

**9.** Procédé selon la revendication 1 ou 2, caractérisé en ce qu'on prépare un composé de formule

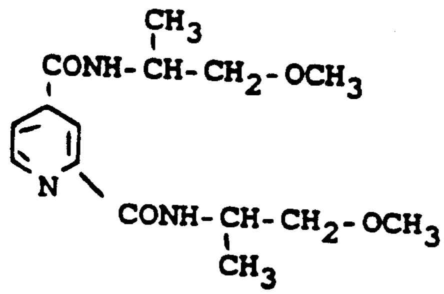

ainsi que ses sels physiologiquement compatibles

**10.** Procédé selon la revendication 1 ou 2, caractérisé en ce qu'on prépare un composé de formule

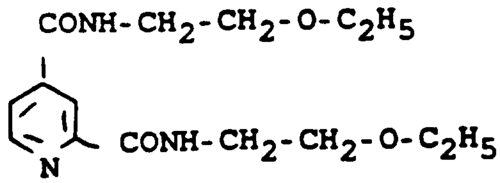

ainsi que ses sels physiologiquement compatibles

**11.** Procédé selon la revendication 1 ou 2, caractérisé en ce qu'on prépare un composé de formule

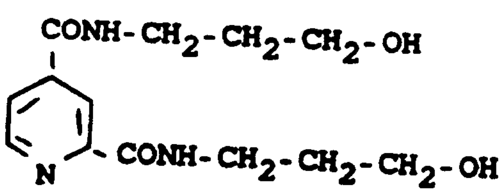

ainsi que ses sels physiologiquement compatibles

**12.** Procédé selon la revendication 1 ou 2, caractérisé en ce qu'on prépare un composé de formule

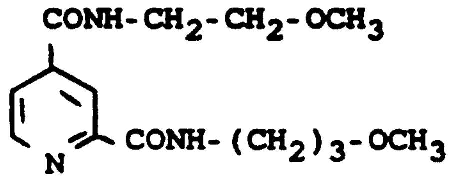

ainsi que ses sels physiologiquement compatibles

**13.** Procédé selon la revendication 1 ou 2, caractérisé en ce qu'on prépare un composé de formule

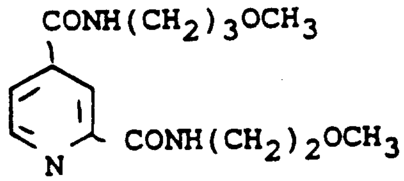

ainsi que ses sels physiologiquement compatibles.

Reaktionsschema